# EUROPEAN PATENT SPECIFICATION

(11) **EP 2 205 219 B1**
(45) Date of publication and mention of the grant of the patent: **02.11.2016**
(21) Application number: 08836498.9
(22) Date of filing: 25.09.2008
(51) Int. Cl.: A61K 9/50, A61K 9/48, A61K 31/337, A61K 31/4375, A61K 31/439, A61K 31/704, A61K 31/728

(54) **METHODS AND FORMULATIONS FOR CONVERTING INTRAVENOUS AND INJECTABLE DRUGS INTO ORAL DOSAGE FORMS**
VERFAHREN UND FORMULIERUNGEN ZUR UMWANDLUNG INTRAVENÖSER UND INJIZIERBARER ARZNEIMITTEL IN ORALE DARREICHUNGSFORMEN
PROCÉDÉS ET FORMULATIONS POUR CONVERTIR DES MÉDICAMENTS INTRAVEINEUX ET INJECTABLES EN DES FORMES DE DOSAGE ORALES

(30) Priority: 28.09.2007 US 864113
(43) Date of publication of application: 14.07.2010
(73) Proprietor: Zomanex, LLC, Chesterfield, MO 63017 (US)
(72) Inventor: SPILBURG, Curtis A.,, Chesterfield Missouri 63017 (US)
(74) Representative: Wood, Graham
(86) International application number: PCT/US2008/077646
(87) International publication number: WO 2009/045837

(56) References cited:
- EP-A- 1 118 333
- EP-A- 1 925 294
- WO-A-99/49848
- WO-A-02/064132
- WO-A-03/094891
- WO-A-2006/095798
- WO-A-2007/096962
- WO-A-2008/046521
- US-A- 5 034 228
- US-A1- 2003 083 313
- US-A1- 2005 019 386
- VELTKAMP S A ET AL: "A novel self-microemulsifying formulation of paclitaxel for oral administration to patients with advanced cancer" BR J CANCER, vol. 95, no. 6, 2006, pages 729-734, XP002509306 cited in the application

## Description

### FIELD OF THE INVENTION

This invention relates to a general method for enhancing the bioavailability of hydrophobic drug active compounds, using naturally-occurring formulation ingredients that are present in the diet. Specifically, this invention is especially useful as a general formulation method for the delivery of drugs in liquid or dry form for oral dosing that heretofore have been administered intravenously or by injection.

### BACKGROUND OF THE INVENTION

Oral drug delivery, the preferred method of administration for most people, remains a subject of intense pharmaceutical and biochemical investigation since the mechanism(s) of drug absorption in the small intestine is largely unknown. It is generally believed that two processes control the amount of drug that is absorbed. First, a high concentration of the active substance at the intestinal membrane surface will enhance cellular absorption (Fick's Law) and, since cells function in an aqueous environment, enhancing the water solubility of a drug increases its concentration at the locus of absorption. However, even though greater water solubility may be expected to enhance the bioavailability of drugs, this is frequently not the case due to a second, competing process that affects the overall absorption process. The absorptive cell membrane is composed mainly of lipids that prevent the passage of hydrophilic water-soluble compounds, but which are highly permeable to lipid soluble substances. Therefore, the design of bioavailable drugs must balance these two opposing forces. On the one hand, a drug that is very hydrophilic may have a high concentration at the cell surface but it may be impermeable to the lipid membrane. On the other hand, a hydrophobic drug that may easily "dissolve" in the membrane lipids may be virtually insoluble in water producing a very low concentration of the active substance at the cell surface. The inherent conflict, for effective oral dosing thus becomes apparent.

The intestinal plasma membrane lines the lumen of the upper gut and is the first absorptive surface to be permeated by most nutrients, foodstuffs and oral dosed drugs. As part of the digestive process, the apical side of the cell is exposed to a complex milieu consisting of pancreatic enzymes, bile and partially digested food from the stomach. Drug absorption does not occur in isolation. Since most drugs are lipophilic, their absorption takes place along with or in competition with that for other lipophilic molecules, such as cholesterol, fat-soluble vitamins, oils and fatty acids. The small intestine is densely covered with villi and microvilli, which greatly enhance the area available for absorption (250 m²), favoring the uptake of even poorly soluble substances. Moreover, the cell surface is also covered with heparin, a negatively charged polysaccharide that tightly binds lipolytic enzymes, such as cholesterol esterase and triglyceride lipase, providing a locus of hydrolytic activity virtually contiguous with the absorptive surface (Bosner MS, et al., Proc Nat 7 Acad Sci 85 : 7438-7442, 1989). This tight binding interaction ensures a high level of lipolytic activity even when the pancreas is not secreting enzymes.

The combination of lipolytic enzymes, bile components and a large intestinal absorption surface provides an environment in which virtually all food is absorbed (Armand M et al., Am J Physiol 271: G172-G183, 1996). While the above-mentioned processes are extremely efficient, the same is not true for certain chemically complex lipids, such as cholesterol, plant sterols, fat soluble vitamins, naturally occurring dietary nutrients, xenobiotics and drugs. Over the past twenty years, much progress has been made in delineating the biochemical processes that are used for the net absorption of these types of compounds, and a central feature of this new understanding is the identification, isolation and dynamic interplay of individual intestinal proteins in the overall absorption process. For drug uptake, the ATP -binding cassette transporter P-glycoprotein (P-gp) plays a pivotal role in modifying the absorption process. Located in high concentration on the villus tip of the apical surface of the brush border membrane, P-gp can serve as a barrier for the intestinal absorption of numerous drug substrates by pumping absorbed drug back into the intestinal lumen (Pang KS, Drug Metab Disp 3J: 1507- 1519, 2005). Thus, increasing the dispersibility of a hydrophobic drug may be thwarted if it is also a substrate of the efflux protein P-gp.

Aqueous dispersibility and susceptibility to small intestinal cell efflux transporters are central problems that therefore must be overcome in order to prepare an oral dosage form for hydrophobic drugs and especially xenobiotics. If these problems cannot be solved then the drug must be given by an alternative methodology, typically intravenously or by injection. These absorption problems are exemplified by (but not limited to) xenobiotics, naturally occurring plant- or marine-derived compounds that have interesting pharmacological properties. Taxanes, camptothecins, anthrocyclines, epipodophyllotoxins, and vinca alkaloids are potent anti-cancer agents that are difficult to formulate in oral dosage forms. To circumvent these delivery problems the oral solid delivery approach is frequently abandoned in favor of an emulsion-based, liquid intravenous strategy. For example, paclitaxel, a potent anti-cancer agent isolated from yew needles, is currently administered intravenously as a dispersion in Cremophor EL, an ethanol blend of castor oil, to create an emulsified paclitaxel dispersion. While this delivery strategy is effective, there are a number of drawbacks that may limit the usefulness of the drug, both from a patient and a biochemical perspective. For example, the intravenous administration occurs in a clinical setting that causes a major disruption in daily activities. This is further complicated by severe hypersensitivity reactions that are the by-product of the Cremophor emulsification system (van Zuylen, L et al., Invest. New Drugs, 2001, 19: 125-141). Because of these vehicle induced problems, patients frequently are premedicated with corticosteroids or histamine antagonists. Finally, because of the dosing method the full therapeutic value of the drug cannot be used. Thus, more frequent dosing would enhance systemic drug levels over time, a result that cannot be achieved with a single intravenous dose that occurs at one, two or three week intervals and is accompanied by non-linear pharmacokinetic behavior (van Tellingen O, Br. J. Cancer, 1999, 81 : 330-335).

Attempts have been made to ameliorate the problems caused by the intravenous, emulsion strategy by simply giving patients the intravenous emulsion orally in the presence of cyclosporine A, a potent inhibitor of small intestinal efflux proteins (Sparreboom A, et al., Proc. Natl Acad Sci, 1997, 94: 2031-2035; Mallingre, MM et al., 2000, J Clin One, 2468-2475). Even though this delivery method has the potential to alleviate at least some of the problems associated with the intravenous method, the presence of Cremophor EL in the oral formulation decreases the overall absorption of paclitaxel (Bardelmeijer, HA et al., 2002, Cancer Chemother Pharmacol 49: 119-125).

Similar to this approach, the pharmaceutical industry has devised a variety of self- emulsifying drug delivery systems that package a drug like paclitaxel in a variety of lipids and surfactants that provide a dispersible matrix when the combination is ingested (Veltkamp SA et al., British J Can, 2006, 95: 729-734). In Veltkamp SA et al, oral paclitaxel dispersed in Cremophor^{®} in the form of a capsule is provided. Cyclosporine A is delivered 30 minutes prior to the dosing of paclitaxel to ensure sufficient time for the cyclosporine A to inhibit the small intestinal P-gp protein before paclitaxel administration. Alternatively, it has been suggested that formulations that are patterned after the lipid composition of digestion phases may provide insight into better ways to solubilize water insoluble drugs (Porter CJH, et al., J Pharm Sci 93: 1110-1121, 2004). While these studies have demonstrated the importance of the digestion process as a guide or template for drug absorption, the approach is empirical requiring exhaustive studies for each drug. Moreover, this strategy is focused more on the physical chemistry of solubilization than on the biochemistry of absorption so it provides little additional insight into the molecular events that are an integral and obligatory part of the absorption process.

Another delivery strategy has been the use of liposomes as an encapsulation vehicle for a variety of drugs for different delivery routes, including oral, parenteral and transdermal (Cevc, G and Paltauf, F., eds., Phospholipids: Characterization, Metabolism, and Novel Biological Applications, pp. 67-79, 126-133, AOCS Press, Champaign, IL, 1995). This method requires amphiphiles, compounds that have a hydrophilic or polar end group and a hydrophobic or non-polar end group, such as phospholipid, cholesterol, glycolipid or a number of food-grade emulsifiers or surfactants. When amphiphiles are added to water, they form lipid bilayer structures (liposomes) that contain an aqueous core surrounded by a hydrophobic membrane. This novel structure can deliver water insoluble drugs that are "dissolved" in its hydrophobic membrane or, alternatively, water soluble drugs can be encapsulated within its aqueous core. This strategy has been employed in a number of fields. For example, liposomes have been used as drug carriers since they are rapidly taken up by the cell and, moreover, by the addition of specific molecules to the liposomal surface they can be targeted to certain cell types or organs, an approach that is typically used for drugs that are encapsulated in the aqueous core. For cosmetic applications, phospholipids and lipid substances are dissolved in organic solvent and, with solvent removal, the resulting solid may be partially hydrated with water and oil to form a cosmetic cream or drug-containing ointment. Finally, liposomes have been found to stabilize certain food ingredients, such as omega-3 fatty acid-containing fish oils to reduce oxidation and rancidity (Haynes et al, U.S. Patent 5,139,803).

Even though liposomes provide an elegant method for drug delivery, their use has been limited by cumbersome preparation methods, inherent instability of aqueous preparations and low drug loading capacity for solid, oral preparations. The utility of a dried preparation to enhance the stability and shelf life of the liposome components has long been recognized, and numerous methods have been devised to maintain the stability of liposomal preparations under drying conditions: Schneider (U.S. Patent 4,229,360); Rahman et al. (4,963,362); Vanlerberghe et al. (U.S. Patent 4,247,411); Payne et al. (U.S. Patents 4,744,989 and 4,830,858). The goal of all these patented methods is to produce a solid that can be re-hydrated at a later time to form liposomes that can deliver a biologically active substance to a target tissue or organ.

Surprisingly, there have been only two reports that use the dried liposome preparations themselves, with no intermediate hydration, as the delivery system. Ostlund, U.S. Patent 5,932,562 teaches the preparation of solid mixes of plant sterols for the reduction of cholesterol absorption. Plant sterols or plant stanols are premixed with lecithin or other amphiphiles in organic solvent, the solvent removed and the solid added back to water and homogenized. The emulsified solution is dried and dispersed in foods or compressed into tablets or capsules. In this case, the active substance is one of the structural components of the liposome itself (plant sterol) and no additional biologically active substance was added. Manzo et al. (U.S. Patent 6,083,529) teach the preparation of a stable dry powder by spray drying an emulsified mixture of lecithin, starch and an anti-inflammatory agent. When applied to the skin, the biologically active moiety is released from the powder only in the presence of moisture. Neither Ostlund nor Manzo suggest or teach the use of sterol, and lecithin and a drug active, all combined with a non-polar solvent and then processed to provide a dried drug carrying liposome of enhanced delivery rates.

Substances other than lecithin have been used as dispersing agents. Following the same steps (dissolution in organic solvent, solvent removal, homogenization in water and spray drying) as those described in U.S. Patent 5,932,562, Ostlund teaches that the surfactant sodium steroyl lactylate can be used in place of lecithin (U.S. Patent 6,063,776). Burruano et al. (U.S. Patents 6,054,144 and 6,1 10,502) describe a method of dispersing soy sterols and stanols or their organic acid esters in the presence of a mono-functional surfactant and a poly-functional surfactant without homogenization. The particle size of the solid plant-derived compounds is first reduced by milling and then mixed with the surfactants in water. This mixture is then spray dried to produce a solid that can be readily dispersed in water. Similarly, Bruce et al. (U.S. Patent 6,242,001) describe the preparation of melts that contain plant sterols/stanols and a suitable hydrocarbon. On cooling these solids can be milled and added to water to produce dispersible sterols. Importantly, none of these methods anticipate the type of delivery method described here as a means to deliver hydrophobic, biologically active compounds.

None of the previous art suggests or teaches methods to enhance the uptake of a drug(s)/sterol/amphilphile combination at a drug loading capacity that would lead to a commercially viable drug delivery system. The stability and ultimate use of liposomal preparations have been shown to depend on the ratio of lecithin to the sterol drug combination. Thus, in order to form creams and parenteral liposomal preparations, previous work focused on the preparation of dispersions containing small liposomal particles (less than 1 µm) by maintaining a high ratio of lecithin to the other components. This prejudice was shown by the requirement that the sum of the drug and the sterol present should not exceed about 25% and preferably about 20% of the total lipid phase present. Hence, the previous art teaches a ratio of lecithin to the sum of the sterol and drug components of at least 3.0, and preferably 4.0 [Perrier et al., U.S. Patent 5,202,126 (c2, line 45), Meybeck & Dumas, U.S. Patent 5,290,562 (c3, line 29)]. Moreover, the purpose of this requirement was to maintain liposomal "quality," which was achieved with a small particle size in order to enhance the stability of the dispersion for the intended uses contained therein [Perrier et al., U.S. Patent 5,202,126 (c4, line 61)]. Departure from this preferred ratio produced sediment which "detracts from the stability of the liposomes" [Perrier et al., U.S. Patent 5,202,126, (c5, line 10)].

In contrast, for the preparation of oral dosage forms it was shown that a superior preparation contained a ratio of the sterol drug combination to amphiphile of 0.2 to 3.0. (Spilburg, patent application, S.N. 11/291,126 , November 30, 2005). This combination produces a delivery system with the following useful and novel advantages: a dispersed solution that can be dried and re-hydrated to produce a dispersion of particles that is similar to that of the dispersion from which it was derived; high drug(s) loading capacity by minimizing the amount of amphiphile in the mix; an emulsion that is stable to conventional drying methods without the addition of large amounts of stabilizers. The dried solid so manufactured can be easily compacted in a tablet and capsule to render the hydrophobic drug bioavailable on ingestion and easily deliverable in a pharmaceutical format.

Moreover, while the previous work of my earlier application focused on the delivery of drugs that were either solids or oils, this present invention extends the utility of this method to show that the method is sufficiently robust to allow for the delivery of drugs - one that provides the proposed therapeutic benefit and one that blocks the action of small intestinal efflux proteins - to provide improved bioavailability. As a result even some cancer drugs like Paclitaxel can now be delivered orally. All of the above described liposome-related art, either deals with cholesterol lowering or with a variety of techniques used in an attempt to solubilize some hydrophobic drugs using specific lipids. None teach or suggest a generalized approach to address the two problems associated with hydrophobic, and especially xenobiotic drug uptake - lack of water dispersibility and interaction with small intestinal cell drug exporters, such as P-gp. An object of the invention is to enhance the biological activity of a hydrophobic drug substance in an oral dosage form through the use of a combination of amphiphiles, surfactants or emulsifiers and a second drug-like substance that blocks small intestinal drug exporters, such as P-gp.

A further object is to provide new oral dosage formulations that can be used for many cancer chemotherapeutics that are naturally occurring chemically complex molecules.

A still further object is to develop a new oral dose form for Paclitaxel. The method of accomplishing these as well as other objectives will become apparent from the detailed description.

### BRIEF DESCRIPTION OF THE DRAWINGS

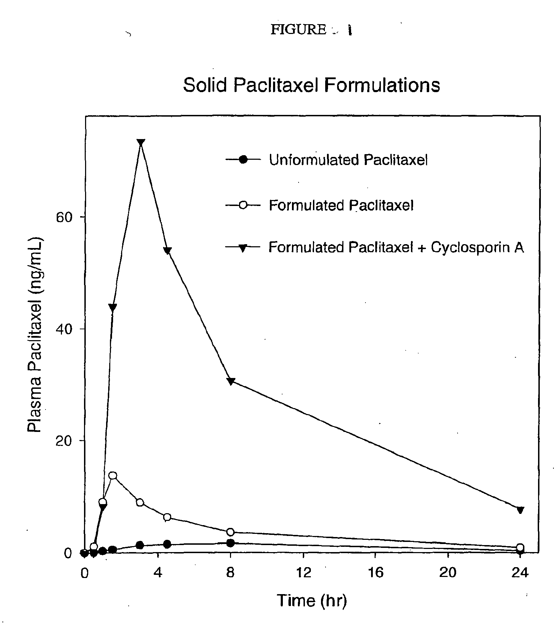

Figure 1 shows the absorption of paclitaxel in female dogs using the solid formulation systems described in Example 1.

### SUMMARY OF THE INVENTION

According to an aspect of the present invention there is provided a composition for the oral delivery of a xenobiotic selected from the group consisting of Taxanes, Camptothecins, Anthrocyclins, Vinca Alkaloids and Epipodophyllotoxins according to claim 1.

According to a further aspect of the present invention there is provided a method of preparing an oral xenobiotic delivery composition in the form of a tablet or capsule, wherein the xenobiotic is selected from the group consisting of Taxanes, Camptothecins, Anthrocyclins, Vinca Alkaloids and Epipodophyllotoxins according to claim 9.

The compositions and methods are provided herein for enhancing the bioavailability of hydrophobic, poorly water soluble compounds and drugs. The compositions contain at least four components - an emulsifier or amphiphile; a sterol (preferably plant-derived); a hydrophobic active or drug compound; and an inhibitor of the small intestinal drug efflux protein. The compositions are especially useful for cancer Chemotherapeutics.

### DETAILED DESCRIPTION OF A PREFERRED EMBODIMENT

There are at least two ways to use the delivery system of this invention. In Method I, the four ingredients are mixed together and processed to provide a single capsule dose. This is a good system but it delivers the drug and the efflux inhibitor at the same time, which may not be optimal for some cases. The second way (Method II) allows for the separate preparation of the active drug and the efflux inhibitor and then dosing them in the same capsule. This allows for each component to be prepared with a different emulsification system that allows the efflux inhibitor to be dispersed more rapidly than the active drug.

### Method I

(a) An amphiphile, such as lecithin or one of its derivatives, a sterol (preferably a plant-derived sterol), the active drug substance and an inhibitor of the drug efflux protein are mixed in a non-polar solvent (preferably ethyl acetate or heptane) at its boiling point.
(b) A solid is collected after the solvent is driven off at elevated temperature to maintain the solubility of all the components.
(c) The solid is broken into small pieces and dispersed with vigorous stirring in water at a temperature that is less than the decomposition temperature of one of the components or the boiling point of water, whichever is lower.
(d) The milky solution is passed through a Gaulin Dairy Homogenizer (or suitable equivalent) operating at maximum pressure; and thereafter
(e) The milky solution is spray dried or lyophilized to produce a solid that can be incorporated into tablets or capsules, providing the appropriate excipients are added.

Optionally, a suitable drying aid is added (Maltrin, Capsule M or suitable equivalent) to assist the drying process.

### Method II

The active drug substance and an inhibitor of the drug efflux protein are prepared separately as described in Method I. The two spray dried powders are then dry blended together and delivered in a single tablet or capsule.

Lecithin is not only an excellent emulsifier and surfactant, it also has many health benefits that are beneficial when used as the contemplated pharmaceutical formulation agent described here [Cevc, G. and Paltauf, F., eds., Phospholipids: Characterization, Metabolism, and Novel Biological Applications, pp. 208-227 AOCS Pres, Champaign, IL, 1995]. While many grades and forms are available, de-oiled lecithin produces the most consistent results. Typical commercially available examples are Ultralec P, Ultralec F and Ultralec G (Archer Daniels Midland, Decatur, IL) or Solec 8160, a powdered, enzyme-modified lecithin (Solae, St. Louis, MO).

Plant-derived sterols, especially those derived from soy and tall oil, are the preferredchoice since they have been shown to lower LDL-cholesterol and they are considered to be safe (Jones PJH et al., Can J. Physiol Pharmacol 75: 227- 235, 1996). Specifically, this invention contemplates the use of mixtures including, but not limited to sitosterol, campesterol, stigmasterol and brassicasterol prepared as described elsewhere (Wester I., et al., "Stanol Composition and the use thereof", WO 98/06405). The reduced forms of the above-mentioned sterols are the most preferred, since they also lower human LDL- cholesterol and their absorption is from five- to tenfold less than that of their non-reduced counterparts (Ostlund RE et al., Am. J. of Physiol, 282: E 911-E916, 2002; Spilburg C et al., J Am Diet Assoc JO3: 577-581, 2003).

A number of criteria can be used to determine appropriate candidates for this formulation system, including but not limited to the following: drugs or organic compounds that are known to be poorly dispersible in water, leading to long dissolution times or; drugs or organic compounds that are known to produce a variable biological response from dose to dose or; drugs that are oils that are difficult to deliver in a conventional tablet or capsule delivery system or; drugs or organic compounds that have been shown to be preferentially soluble in hydrophobic solvent as evidenced by their partition coefficient in the octanol water system or; drugs that are preferentially absorbed when consumed with a fatty meal or; drugs that can only be delivered intravenously or by injection. In addition to these components, other ingredients may be added that provide beneficial properties to the final product, such as vitamin E to maintain stability of the active species.

Inhibitors of the small intestinal efflux protein or of cytochrome P450 include, but are not limited to, verapamil, cyclosporin A, cyclosporine D, erythromycin, quinine, fluphenazine, reserpine, progesterone, tamoxifen, mitotane, annamycin, biricodar, elacridar, tariquidar and zosuquidar.

For those drugs that are compatible with organic solvents, all the formulation components are dissolved in a suitable non-polar organic solvent, such as chloroform, dichloromethane, ethyl acetate, pentane, hexane, heptane or supercritical carbon dioxide. The choice of solvent is dictated by the solubility of the components and the stability of the drug at the temperature of the solvent. The preferred solvents are non-chlorinated and for heat stable compounds, heptane is the most preferred solvent because of its high boiling point, which increases the overall solubility of all the components.

The weight fraction of each component in the final four-component mixture depends on the nature of the hydrophobic compound(s), the nature of the emulsifier amphiphile used to prepare the blend and the intended use of the final product - tablet, capsule, food product or beverage. Regardless of method, the goal is to produce an emulsified mixture of drug, inhibitor of the efflux protein, sterols and amphiphile so that the amount of amphiphile in the system is minimized relative to the other components. To achieve this end for Method I, in the total blend containing all four components, the weight fraction of each component is given in the table below.

| FRACTION BY WEIGHT OF EACH COMPONENT IN THE FINAL BLEND | | |
|---|---|---|
| Component | Broad Range | Preferred Range |
| Amphiphile (emulsifier) | 0.075 - 0.95 | 0.20-0.80 |
| Sterol | 0.02 - 0.75 | 0.10-0.60 |
| Drug active effective amt. | 0.02 - 0.50 | 0.10-0.40 |
| Intestinal efflux inhibitor | 0.012-0.50 | 0.10-0.40 |

The ranges described in the table above also apply for Method II. However, for this method the active drug and the inhibitor of the efflux protein are prepared separately, but when they are combined together in the same capsule or in separate capsules, the ranges above still apply. Importantly, in all methods, sufficient amphiphile must be present to allow dispersibility.

After all the components are dissolved at the desired ratio in the appropriate solvent, the liquid is removed at elevated temperature to maintain the solubility and stability of all the components. Residual solvent can be removed by pumping under vacuum. Alternatively, the solvent can be removed by atomization as described in U.S. Patents 4,508,703 and 4,621,023. The solid is then added to water at a temperature that is less than the decomposition temperature of one of the components or the boiling point of water, whichever is lower. The mixture is vigorously mixed in a suitable mixer to form a milky solution, which is then homogenized, preferably with a sonicator, Gaulin dairy homogenizer or a microfluidizer. The water is then removed by spray drying, lyophilization or some other suitable drying method. Before drying, it is helpful but not necessary, to add maltrin, starch, silicon dioxide, calcium silicate or sodium croscarmellose to produce a flowable powder that has more desirable properties for filling capsules, compression into tablets or addition to certain medical foods. The addition of a suitable antacid, such as calcium carbonate or the like, to the powder at a weight per cent of 0.5 to 10.0 stabilizes and/or activates the components in the blend to produce a superior product. For some blends, either wet or solid granulation produces a superior solid with a greater bulk density. The dried liposomal blend described above is the starting point for a variety of flexible delivery systems described below. Since the key components of the powdered formulation system are compounds that are an integral result of the digestive process, they are compatible with food delivery systems that can be especially designed for children and the elderly. The powdered drug/plant sterol/lecithin blend described above can be easily dispersed in milk or other beverages for convenient delivery to neonates and infants. Moreover, the absence of pancreatic lipolytic activity and low concentrations of bile salt are not an impediment to drug absorption since the drug is packaged in a system that contains components that are the end product of the digestive process. This is of special importance for neonates and adults with pancreatic insufficiency, such as cystic fibrosis patients. In summary, the proposed formulation system provides a seamless transition from neonates - powder dispersed in milk - to children - powder compressed in a chewable tablet - to adults - powder compressed in a conventional tablet or capsules.

The precise details of tableting technique are not a part of this invention, and since they are well-known they need not be described herein in detail.

While not precisely knowing why, and not wishing to be bound by any theory of operability, the fact is that for difficulty soluble drugs this composition and combination of steps achieved higher absorption and lower variability of absorption.

In the examples to follow, the novelty and utility of the method will be shown in a solid delivery system. The improvement in the uptake will be shown by comparing the formulation system to that available in the corresponding commercially available unformulated drug. To these ends, pharmacokinetic studies were performed in five naïve, female beagle dogs with each drug dosed in a formulation system using a crossover design, with a one week wash out period between doses. All animal work was performed following procedures for animal care and housing that were in accordance with the Guide for the Care and Use of Laboratory Animals (Institute of Laboratory Animal Resources, Commission on Life Sciences, National Research Council, National Academic Press, 1996). Following a 16-hour fast, the animals were fed a small amount (approximately 1/4 can) of Hill/s Science Diet A/D and thirty minutes later each animal was orally dosed with one of the formulations of the appropriate test article. Blood samples were drawn 0.5, 1.0, 1.5, 3.0, 4.5, 8.0, and 24 hours after dosing.

### EXAMPLE 1

A solid formulation method was also used to determine the effect of the formulation system in the presence or absence of cyclosporin A (P-gp inhibitor).

Solid Preparation - Paclitaxel. Solid Paclitaxel (300 mg), soy sterols (300 mg) and lysolecithin (900 mg) were added to a 30 mL glass tube and chloroform (3.0 ml) was added. After the solids were dissolved with gentle heating in a 60°C water bath, the solvent was removed under a stream of nitrogen. The mass was then pumped on under vacuum to remove residual solvent. Addition of water (15 mL) softened the solid mass and the mixture was then sonicated in an ice bath for two minutes on 40% power, followed by two minutes sonication on 50% power and then two minutes sonication on 60% power. The milky solution was then transferred to a lyophilization jar and croscarmellose and fumed silica were added followed by an additional two-minute period of sonication at 60% power to disperse the solids. The milky solution was then shell frozen in a dry ice-acetone bath and lyophilized. Lyophilized formulated Paclitaxel (110 mg, 21 mg Paclitaxel) was dry granulated with calcium carbonate, Maltrin^{®} and silicon dioxide. There was a noticeable decrease in the bulk density and the flowable powder was packed into a "000" capsule. This granulation process was repeated five times for five separate capsules.

Solid Preparations - Formulated Paclitaxel + Cyclosporin A. Solid cyclosporin A (500 mg), soy sterols (500 mg) and lecithin (1000 mg) were added to each of two 30 mL glass tubes and chloroform (3.0 ml) was added. A lyophilized blend of the components was prepared as described above for solid Paclitaxel. To increase the bulk density of the cyclosporin blend, the powder was wet granulated with calcium carbonate by spraying with 10% polyvinylpyrrolidone dissolved in 91% isopropanol. The blend was set aside to air dry for 48 hours and the pale yellow solid was collected and passed through a #10 screen. Larger granules were milled in a coffee grinder and the solid was re-screened. Capsules were filled in two steps. First, cyclosporin granules were weighed into a "000" capsule and allowed to stand in an upright position with the cap not installed. Second, dry granulated Paclitaxel was then added, and the capsule head was firmly installed.

Control Experiment - Solid Unformulated Paclitaxel. Calcium carbonate (50 mg), Maltrin^{®} (75 mg) and silicon dioxide (3 mg) were weighed and added to a "000" gelatin capsule. In a separate weighing, Paclitaxel (20 mg) was weighed and added to the other ingredients in the capsule. The capsule cap was installed to the bottom piece and the contents were vigorously shaken to blend the solids.

Absorption Experiment With Solid Formulations. After dosing with each formulation, all the blood samples were processed and analyzed as described for the liquid formulations. As shown in Figure 2, there is a marked increase in Paclitaxel absorption for the two solid formulations when compared to that for the unformulated Paclitaxel. To quantitate the absorption changes, the area under the curve (AUC_{o→∞}) was calculated for each formulation system and the results are shown in the Table below. Compared to the unformulated Paclitaxel, there was a statistically significant 3.5-fold (p = 0.02) increase in absorption from the formulation system alone, and a 26-fold (p = 0.008) increase when compared to the formulated Paclitaxel cyclosporin A combination.

| EFFECT OF SOLID FORMULATION ON PACLITAXEL UPTAKE | |
|---|---|
| Formulation | AUC_{o→∞} (ng/mLh⁻¹) |
| (A)Unformulated Paclitaxel | 28.9 ± 7.1 |
| (B)Formulated Paclitaxel | 101.2 ± 23.4 |
| (C)Formulated Paclitaxel plus Cyclosporin A | 752.1 ± 134.5 |

| | |
|---|---|
| A vs B, p = 0.02, A vs C, p = 0.005; B vs C, p = 0.008 | |

The experiment indicates that improved paclataxel absorption occurs when the xenobiotic is formulated in a sterol emulsifier combination, which is designed to enhance its dispersibility in the small intestinal lumen. Even though this produces an impressive 3.5 - 4.0-fold increase in absorption when compared to that of the unformulated solid, the small intestinal efflux transporter expels much of the absorbed drug. The addition of an inhibitor of the export protein (cyclosporine A), formulated in the same system as that used for Paclitaxel, increases the absorption 25-40-fold relative to that for the unformulated solid, demonstrating that optimum absorption occurs when the exporter is inhibited and when the hydrophobic components are in a dispersible formulation. To my knowledge, this is the first demonstration that Paclitaxel can be efficiently absorbed as a solid.

The above described examples are illustrative of the invention, which is of course broader than the specific examples. The scope of the invention is defined by the appended claims.

## Claims

1. A composition for the oral delivery of a xenobiotic selected from the group consisting of Taxanes, Camptothecins, Anthrocyclins, Vinca Alkaloids and Epipodophyllotoxins , said composition comprising:
component a) of a spray dried or lyophilized powder of the xenobiotic;
lecithin; and
a plant-derived sterol; and
a component b) a spray dried or lyophilized powder of an effective amount of an inhibitor of small intestinal efflux proteins;
lecithin; and
a plant-derived sterol,
wherein said component a) and b) are prepared separately, are dry blended together, and are packed into a single capsule or a single tablet.

2. The composition of claim 1, wherein the xenobiotic is a Taxane.

3. The composition of claim 2, wherein the xenobiotic is paclitaxel.

4. The composition of claim 1 wherein the plant derived sterol is derived from a vegetable or tall oil source.

5. The composition of claim 1, wherein the inhibitor of small intestinal efflux proteins is cyclosporine A.

6. The composition of claim 1 wherein the lecithin is from 7.5% by weight to 95% by weight of the composition; the plant-derived sterol is from 2% by weight to 75% by weight of the composition; the xenobiotic is from 2% to 50% by weight of the composition; and, the small intestine efflux inhibitor is from 2% to 50% by weight of the total composition.

7. The composition of claim 6 wherein the lecithin is from 20% by weight to 80% by weight of the composition; the plant derived sterol is from 10% by weight to 60% by weight of the composition; the xenobiotic is from 10% to 40% by weight of the composition; and, the small intestine efflux inhibitor is from 10% to 40% by weight of the total composition.

8. The composition of claim 1 wherein the small intestine efflux inhibitor is selected from the group consisting of verapamil, cyclosporin A, cyclosporine D, erythromycin, quinine, fluphenazine, reserpine, progesterone, tamoxifen, mitotane, annamycin, biricodar, elacridar, tariquidar and zosuquidar.

9. A method of preparing an oral xenobiotic delivery composition in the form of a tablet or capsule, wherein the xenobiotic is selected from the group consisting of Taxanes, Camptothecins, Anthrocyclins, Vinca Alkaloids and Epipodophyllotoxins, said method comprising the steps:
a) mixing together the following:
the xenobiotic;
lecithin;
a plant derived sterol; and
a non-polar solvent at its boiling point; and
removing the solvent at elevated temperature to maintain the solubility of all the components to leave a solid residue of the mixed components;
breaking the solid into small pieces and dispersing the same in water at a temperature less than the decomposition temperature of any one of the mixed components or the boiling point of water, whichever is lower, to yield a milky solution;
homogenizing the milky solution using a homogenizer operating at maximum pressure; spray drying or lyophilizing the milky solution to produce a solid;
b) mixing together the following:
an effective amount of an inhibitor of small intestine efflux proteins;
lecithin;
a plant derived sterol; and
a non-polar solvent at its boiling point; and
removing the solvent at elevated temperature to maintain solubility of all the components to leave a solid residue of the mixed components;
breaking the solid into small pieces and dispersing the same in water at a temperature less than the decomposition temperature of any one of the mixed components or the boiling point of water, whichever is lower, to yield a milky solution;
homogenizing the milky solution using a homogenizer operating at maximum pressure; spray drying or lyophilizing the milky solution to produce a solid; and
dry blending components a) and b) for delivery in a single capsule or single tablet.

10. The method of claim 9 wherein the non-polar organic solvent is selected from the group consisting of ethyl acetate, chloroform, dichloromethane, super critical carbon dioxide and heptane.

11. The method of claim 9 wherein the small intestine efflux inhibitor is selected from the group consisting of verapamil, cyclosporin A, cyclosporine D, erythromycin, quinine, fluphenazine, reserpine, progesterone, tamoxifen, mitotane, annamycin, biricodar, elacridar, tariquidar and zosuquidar.

## Patentansprüche

1. Zusammensetzung zur oralen Zuführung eines Xenobiotikums, ausgewählt aus der Gruppe bestehend aus Taxanen, Camptothecinen, Anthrocyclinen, Vinca-Alkaloiden und Epipodophyllotoxinen, wobei die genannte Zusammensetzung Folgendes umfasst:
Komponente a) eines sprühgetrockneten oder lyophilisierten Pulvers des Xenobiotikums;
Lecithin; und
ein pflanzliches Sterol; und
eine Komponente b) eines sprühgetrockneten oder lyophilisierten Pulvers einer wirksamen Menge eines Inhibitors von Dünndarmausfluss-Proteinen;
Lecithin; und
ein pflanzliches Sterol,
wobei die genannten Komponenten a) und b) separat hergestellt, im trockenen Zustand miteinander vermischt und zu einer einzigen Kapsel oder einer einzigen Tablette verpackt werden.

2. Zusammensetzung nach Anspruch 1, wobei das Xenobiotikum ein Taxan ist.

3. Zusammensetzung nach Anspruch 2, wobei das Xenobiotikum Paclitaxel ist.

4. Zusammensetzung nach Anspruch 1, wobei das pflanzliche Sterol von einer Pflanzen- oder Tallölquelle gewonnen wurde.

5. Zusammensetzung nach Anspruch 1, wobei der Inhibitor von Dünndarmausfluss-Proteinen Cyclosporin A ist.

6. Zusammensetzung nach Anspruch 1, wobei das Lecithin 7,5 Gew.-% bis 95 Gew.-% der Zusammensetzung ausmacht; das pflanzliche Sterol 2 Gew.-% bis 75 Gew.-% der Zusammensetzung ausmacht; das Xenobiotikum 2 bis 50 Gew.-% der Zusammensetzung ausmacht und der Dünndarmausfluss-Inhibitor 2 bis 50 Gew.-% der Gesamtzusammensetzung ausmacht.

7. Zusammensetzung nach Anspruch 6, wobei das Lecithin 20 Gew.-% bis 80 Gew.-% der Zusammensetzung ausmacht; das pflanzliche Sterol 10 Gew.-% bis 60 Gew.-% der Zusammensetzung ausmacht; das Xenobiotikum 10 bis 40 Gew.-% der Zusammensetzung ausmacht und der Dünndarmausfluss-Inhibitor 10 bis 40 Gew.-% der Gesamtzusammensetzung ausmacht.

8. Zusammensetzung nach Anspruch 1, wobei der Dünndarmausfluss-Inhibitor ausgewählt ist aus der Gruppe bestehend aus Verapamil, Cyclosporin A, Cyclosporin D, Erythromycin, Chinin, Fluphenazin, Reserpin, Progesteron, Tamoxifen, Mitotan, Annamycin, Biricodar, Elacridar, Tariquidar und Zosuquidar.

9. Verfahren zur Herstellung einer oralen Xenobiotikum-Zuführungszusammensetzung in Form einer Tablette oder einer Kapsel, wobei das Xenobiotikum ausgewählt ist aus der Gruppe bestehend aus Taxanen, Camptothecinen, Anthrocyclinen, Vinca-Alkaloiden und Epipodophyllotoxinen, wobei das genannte Verfahren die folgenden Schritte beinhaltet:
a) Mischen der folgenden Bestandteile miteinander:
das Xenobiotikum;
Lecithin;
ein pflanzliches Sterol; und
ein nicht-polares Lösungsmittel an seinem Siedepunkt; und
Entfernen des Lösungsmittels bei einer erhöhten Temperatur, um die Löslichkeit aller Komponenten zu erhalten, um einen festen Rückstand der gemischten Komponenten zu hinterlassen;
Zerbrechen des Feststoffs in kleine Stücke und Dispergieren derselben in Wasser bei einer Temperatur unter der Zersetzungstemperatur einer beliebigen der gemischten Komponenten oder dem Siedepunkt von Wasser, was immer niedriger ist, um eine milchige Lösung zu erhalten;
Homogenisieren der milchigen Lösung mit einem Homogenisator, der bei maximalem Druck arbeitet;
Sprühtrocknen oder Lyophilisieren der milchigen Lösung, um einen Feststoff zu erzeugen;
b) Mischen der folgenden Bestandteile miteinander:
eine wirksame Menge eines Inhibitors von Dünndarmausfluss-Proteinen;
Lecithin;
ein pflanzliches Sterol; und
ein nicht-polares Lösungsmittel an seinem Siedepunkt; und
Entfernen des Lösungsmittels bei erhöhter Temperatur, um die Löslichkeit aller Komponenten zu erhalten, um einen festen Rückstand der gemischten Komponenten zu hinterlassen;
Zerbrechen des Feststoffs in kleine Stücke und Dispergieren derselben in Wasser bei einer Temperatur unter der Zersetzungstemperatur von einer beliebigen der gemischten Komponenten oder dem Siedepunkt von Wasser, was immer niedriger ist, um eine milchige Lösung zu erhalten;
Homogenisieren der milchigen Lösung mit einem Homogenisator, der auf maximalem Druck arbeitet;
Sprühtrocknen oder Lyophilisieren der milchigen Lösung, um einen Feststoff zu erzeugen; und
Mischen der Komponenten a) und b) im trockenen Zustand zur Lieferung in einer einzigen Kapsel oder einer einzigen Tablette.

10. Verfahren nach Anspruch 9, wobei das nicht-polare organische Lösungsmittel ausgewählt ist aus der Gruppe bestehend aus Ethylacetat, Chloroform, Dichlormethan, überkritischem Kohlenstoffdioxid und Heptan.

11. Verfahren nach Anspruch 9, wobei der Dünndarmausfluss-Inhibitor ausgewählt ist aus der Gruppe bestehend aus Verapamil, Cyclosporin A, Cyclosporin D, Erythromycin, Chinin, Fluphenazin, Reserpin, Progesteron, Tamoxifen, Mitotan, Annamycin, Biricodar, Elacridar, Tariquidar und Zosuquidar.

## Revendications

1. Composition pour la libération orale d'un xénobiotique sélectionné dans le groupe constitué de taxanes, de camptothécines, d'anthrocyclines, de vinca-alcaloïdes et d'épidodophyllotoxines, ladite composition comprenant :
un composant constitué a) d'une poudre séchée par pulvérisation ou lyophilisée du xénobiotique ;
de lécithine ; et
d'un stérol dérivé de plantes ; et
un composant constitué b) d'une poudre séchée par pulvérisation ou lyophilisée contenant une quantité efficace d'un inhibiteur des protéines d'efflux de l'intestin grêle ;
de lécithine ; et
d'un stérol dérivé de plantes ; et
dans laquelle lesdits composants a) et b) sont préparés séparément, sont mélangés à sec ensemble, et sont conditionnés dans une gélule unique ou un comprimé unique.

2. Composition de la revendication 1, dans lequel le xénobiotique est un taxane.

3. Composition de la revendication 2, dans laquelle le xénobiotique est le paclitaxel.

4. Composition de la revendication 1 dans laquelle le stérol dérivé de plantes est dérivé d'une source végétale ou d'une source d'huile de tall.

5. Composition de la revendication 1, dans laquelle l'inhibiteur des protéines d'efflux de l'intestin grêle est la cyclosporine A.

6. Composition de la revendication 1 dans laquelle la lécithine représente de 7,5 % en poids à 95 % en poids de la composition ; le stérol dérivé de plantes représente de 2 % en poids à 75 % en poids de la composition ; le xénobiotique représente de 2 % en poids à 50 % en poids de la composition ; et l'inhibiteur d'efflux de l'intestin grêle représente de 2 % à 50 % en poids de la composition totale.

7. Composition de la revendication 6 dans laquelle la lécithine représente de 20 % à 80 % en poids de la composition ; le stérol dérivé de plantes représente de 10 % en poids à 60 % en poids de la composition ; le xénobiotique représente de 10 % à 40 % en poids de la composition ; et, l'inhibiteur d'efflux de l'intestin grêle représente de 10 % à 40 % en poids de la composition totale.

8. Composition de la revendication 1 dans laquelle l'inhibiteur d'efflux de l'intestin grêle est sélectionné dans le groupe constitué de vérapamil, de cyclosporine A, de cyclosporine D, d'érythromycine, de quinine, de fluphénazine, de réserpine, de progestérone, de tamoxifène, de mitotane, d'annamycine, de biricodar, d'élacridar, de tariquidar et de zosuquidar.

9. Procédé de préparation d'une composition de libération de xénobiotique sous forme de comprimé ou de gélule, dans lequel le xénobiotique est sélectionné dans le groupe constitué de taxanes, de camptothécines, d'anthrocyclines, de vinca-alcaloïdes et d'épipodophyllotoxines, ledit procédé comprenant les étapes consistant à :
a) mélanger ensemble ce qui suit :
le xénobiotique ;
la lécithine ;
un stérol dérivé de plantes ; et
un solvant non polaire à son point d'ébullition ; et
éliminer le solvant à une température élevée afin de maintenir la solubilité de tous les composants dans le but de laisser un résidu solide des composants mélangés ;
fractionner le solide en parties plus petites et les disperser dans de l'eau à une température inférieure à la température de décomposition de l'un quelconque des composants mélangés ou du point d'ébullition de l'eau, si ce dernier est inférieur, afin de produire une solution laiteuse ;
homogénéiser la solution laiteuse en utilisant un homogénéisateur fonctionnant à la pression maximale ;
sécher par pulvérisation ou lyophiliser la solution laiteuse afin de produire un solide ;
b) mélanger ensemble ce qui suit :
une quantité efficace d'un inhibiteur des protéines d'efflux de l'intestin grêle ;
de la lécithine ;
un stérol dérivé de plantes ; et
un solvant non polaire à son point d'ébullition ; et
éliminer le solvant à une température élevée pour maintenir la solubilité des tous les composants dans le but de laisser un résidu solide des composants mélangés ;
fractionner le solide en parties plus petites et les disperser dans de l'eau à une température inférieure à la température de décomposition de l'un quelconque des composants mélangés ou du point d'ébullition de l'eau, si ce dernier est inférieur, afin de produire une solution laiteuse ;
homogénéiser la solution laiteuse en utilisant un homogénéisateur fonctionnant à la pression maximale ;
sécher par pulvérisation ou lyophiliser la solution laiteuse afin de produire un solide ; et
mélanger à sec les composants a) et b) pour un mode d'administration en gélule unique ou en comprimé unique.

10. Procédé de la revendication 9 dans lequel le solvant organique non polaire est sélectionné dans le groupe constitué d'acétate d'éthyle, de chloroforme, de dichlorométhane, de dioxyde de carbone super-critique et d'heptane.

11. Procédé de la revendication 9 dans lequel l'inhibiteur d'efflux de l'intestin grêle est sélectionné dans le groupe constitué de vérapamil, de cyclosporine A, de cyclosporine B, d'érythromycine, de quinine, de fluphénazine, de réserpine, de progestérone, de tamoxifène, de mitotane, d'annamycine, de biricodar, d'élacridar, de tariquidar et de zosuquidar.
